(19) Europäisches Patentamt
European Patent Office
Office européen des brevets

(11) **EP 2 491 956 A1**

(12) **EUROPEAN PATENT APPLICATION**
published in accordance with Art. 153(4) EPC

(43) Date of publication:
**29.08.2012 Bulletin 2012/35**

(51) Int Cl.:
*A61L 2/16* *(2006.01)*     *A61L 9/00* *(2006.01)*
*A61L 9/01* *(2006.01)*     *A61L 9/18* *(2006.01)*
*A61L 9/20* *(2006.01)*     *B01J 35/02* *(2006.01)*

(21) Application number: **10824917.8**

(22) Date of filing: **19.10.2010**

(86) International application number:
**PCT/JP2010/068333**

(87) International publication number:
**WO 2011/049068 (28.04.2011 Gazette 2011/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.10.2009 JP 2009240680**

(71) Applicants:
• **The University of Tokyo**
  **Bunkyo-Ku**
  **Tokyo 113-8654 (JP)**
• **KANAGAWA ACADEMY OF SCIENCE AND TECHNOLOGY**
  **Kawasaki-shi,**
  **Kanagawa-ken 213-0012 (JP)**
• **Showa Denko K.K.**
  **Tokyo 105-8518 (JP)**

(72) Inventors:
• **HASHIMOTO, Kazuhito**
  **Tokyo 113-8654 (JP)**
• **SUNADA, Kayano**
  **Tokyo 113-8654 (JP)**

• **KUBOTA, Yoshinobu**
  **Kawasaki-shi**
  **Kanagawa 213-0012 (JP)**
• **ISHIGURO, Hitoshi**
  **Kawasaki-shi**
  **Kanagawa 213-0012 (JP)**
• **NAKANO, Ryuichi**
  **Kawasaki-shi**
  **Kanagawa 213-0012 (JP)**
• **KAJIOKA, Jitsuo**
  **Kawasaki-shi**
  **Kanagawa 213-0012 (JP)**
• **YAO, Yanyan**
  **Kawasaki-shi**
  **Kanagawa 213-0012 (JP)**
• **KURODA, Yasushi**
  **Toyama-shi**
  **Toyama 931-8577 (JP)**
• **HOSOGI, Yasuhiro**
  **Toyama-shi**
  **Toyama 931-8577 (JP)**

(74) Representative: **Strehl Schübel-Hopf & Partner**
  **Maximilianstraße 54**
  **D-80538 München (DE)**

(54) **METHOD FOR INACTIVATING VIRUS AND ARTICLE PROVIDED WITH ANTIVIRAL PROPERTIES**

(57)     To provide a method for inactivating a virus which is in contact with a photocatalyst material, the method including irradiating the photocatalyst material with light from a light source, and an article provided with an anti-viral property, the article containing a visible-light-responsive photocatalyst material deposited on the surface thereof.

FIG.1

EP 2 491 956 A1

## Description

Technical Field

The present invention relates to a simple method for inactivating a virus, for the purpose of prevention of infection of an animal and a human with the virus, through utilization of light as a sole energy source, and to an article provided with an anti-viral property containing a visible-light-responsive photocatalyst material on the surface thereof.

Background Art

Photocatalyst materials exhibit an activity of decomposing or oxidizing organic substances and inorganic substances (e.g., nitrogen oxide) through employment of light as an energy source, which is low-cost and very environment-friendly. In recent years, applications of photocatalyst material have been extended to environmental cleaning, deodorization, anti-fouling, and sterilization, etc., and a variety of photocatalyst materials have been developed and studied.

Meanwhile, one of the current concerns in the world is explosive infection with a new-type influenza virus. The photocatalyst is envisaged to provide a technique which can inactivate such a virus and prevent infection therewith.

Some studies have revealed that a photocatalyst can inactivate a virus. However, the action mechanism of the photocatalyst on a virus has not been clearly elucidated. Thus, elucidation of the action mechanism is thought to be a key to development of photocatalyst materials in the future.

Among photocatalyst materials, there is demand for a photocatalyst material which exhibits its activity under irradiation with visible light. In fact, research and development of the materials are currently ongoing. If a technique which can be inactivated a virus under irradiation with visible light is developed, an infection-active virus is expected to be easily reduced under an illumination source of general use (e.g., a fluorescent lamp).

In recent years, visible-light-responsive photocatalyst materials mainly formed of tungsten oxide have been developed. For example, Patent Document 1 discloses that tungsten oxide serves as a useful visible-light-responsive photocatalyst material when used along with a copper compound serving as a catalyst-activity-promoter.

Non-Patent Document 1 discloses that tungsten oxide and titanium oxide each containing copper ions or iron ions serve as useful visible-light-responsive photocatalyst material.

These materials are thought to absorb light of a longer wavelength more effectively as compared with a conventional nitrogen-doped titanium oxide photocatalyst material.

Patent Document 2 discloses an air-cleaning member which can inactivate a virus by the action of a photocatalyst. This technique inactivates a virus under irradiation with UV light by use of a black lamp.

Patent Documents 3 and 4 disclose a photocatalyst which is also responsive to visible light and which contains a titanium oxide sol. In the titanium oxide sol, two titanium oxide species having different crystal grain size and morphologies are linked together via OH groups, and either of the two species is doped with nitrogen. When receiving light, a member containing the photocatalyst can reduce the titer of a virus. Patent Document 5 discloses that a combination of catechin and nitrogen-doped titanium oxide provides an anti-viral activity under visible light.

Prior Art Documents

Patent Documents

Patent Document 1: Japanese Patent Application Laid-Open (*kokai*) No. 2008-149312
Patent Document 2: Japanese Patent No. 3649241
Patent Document 3: Japanese Patent No. 4240505
Patent Document 4: Japanese Patent No. 4240508
Patent Document 5: Japanese Patent Application Laid-Open (*kokai*) No. 2008-119312

Non-Patent Documents

Non-Patent Document 1: Irie et al., Bulletin Photocatalyst, Vol. 28, p. 4, April, 2009

Summary of the Invention

Problems to be Solved by the Invention

According to the technique disclosed in Patent Documents 3 and 4, the virus titer is reduced under a fluorescent lamp. However, absorption of light of a longer wavelength is thought to be difficult in consideration of the structural characteristics of the photocatalyst, and the rate of reduction is small. According to the technique disclosed in Patent Document 5, the employed catechin component has high anti-viral property, and a determination cannot be made on whether or not the anti-viral effect is attributed only to the visible-light-responsive photocatalyst.

The present invention has been conceived under such circumstances. Thus, an object of the present invention is to provide a method for inactivating a virus employing a UV-beam-responsive or visible-light-responsive photocatalyst material, particularly employing a visible-light-responsive photocatalyst material under irradiation with light, wherein the virus is inactivated through irradiation with light including visible light of a wavelength of 400 to 530 nm. Another object of the invention is to provide an article provided with an anti-viral property, which article employs visible light as an energy source.

Means for Solving the Problems

The present inventors have conducted extensive studies in order to attain the aforementioned objects, and have found that a virus which is in contact with a photocatalyst material can be inactivated by irradiating the photocatalyst material with light to partially destroy membrane protein of the virus. The inventors have also found that, through employment of a visible-light-responsive photocatalyst comprising, in combination, a copper compound and/or an iron compound and at least one member selected from among tungsten oxide, titanium oxide, and titanium oxide having a conduction band controlled through doping, an anti-viral performance can be attained under irradiation with visible light, particularly under irradiation with light having a relatively long wavelength of 400 to 530 nm.
The present invention has been accomplished on the basis of these findings.

Accordingly, the present invention provides the following.

[1] A method for inactivating a virus which is in contact with a photocatalyst material, the method comprising irradiating the photocatalyst material with light from a light source.
[2] A virus inactivation method according to [1] above, wherein membrane protein in the virus which is in contact with the photocatalyst material is partially destroyed.
[3] A virus inactivation method according to [1] or [2] above, wherein the photocatalyst material contains titanium oxide, and the light from the light source includes ultraviolet light having a wavelength of 350 to 400 nm.
[4] A virus inactivation method according to [3] above, wherein the photocatalyst material is in the form of coating film, and the coating film contains titanium oxide in an amount of 10 mg/$m^2$ to 10 g/$m^2$.
[5] A virus inactivation method according to [3] or [4] above, wherein the light source provides light having a wavelength of 350 to 400 nm and is any of sunlight, a fluorescent lamp, an LED, and an organic EL.
[6] A virus inactivation method according to [1] or [2] above, wherein the photocatalyst material contains a visible-light-responsive photocatalyst material, and the light from the light source includes visible light having a wavelength of 400 to 530 nm.
[7] A virus inactivation method according to [6] above, wherein the visible-light-responsive photocatalyst material comprises, in combination, (A-1) a copper compound and/or an iron compound, and (B) at least one member selected from among tungsten oxide, titanium oxide, and titanium oxide having a conduction band controlled through doping.
[8] A virus inactivation method according to [6] above, wherein the visible-light-responsive photocatalyst material comprises, in combination, (A-2) any of platinum, palladium, rhodium, and ruthenium, or a mixture of two or more species thereof, and (B) at least one member selected from among tungsten oxide, titanium oxide, and titanium oxide having a conduction band controlled through doping.
[9] A virus inactivation method according to any of [6] to [8] above, wherein the photocatalyst material is in the form of coating film, and the coating film has a visible-light-responsive photocatalyst material content of 100 mg/$m^2$ to 20 g/$m^2$.
[10] A virus inactivation method according to any of [5] to [9] above, wherein the light source provides light having a wavelength of 400 to 530 nm and is any of sunlight, a fluorescent lamp, an LED, and an organic EL.
[11] A virus inactivation method according to any of [1] to [10] above, wherein the virus is an influenza virus.
[12] A virus inactivation method according to any of [1] to [10] above, wherein the virus is a bacteriophage.
[13] An article provided with an anti-viral property, comprising a visible-light-responsive photocatalyst material deposited on a surface thereof.
[14] An article provided with an anti-viral property according to [13] above, wherein the visible-light-responsive photocatalyst material comprises, in combination, (A) a copper compound and/or an iron compound, and (B) at least one member selected from among tungsten oxide, titanium oxide, and titanium oxide having a conduction band controlled through doping.

Effects of the Invention

According to the present invention, there can be provided a method for inactivating a virus employing a UV-light-responsive or visible-light-responsive photocatalyst material under irradiation with light, particularly employing a visible-light-responsive photocatalyst material under irradiation with light including visible light of a wavelength of 400 to 530 nm, as well as an article provided with an anti-viral property, which article employs visible light as an energy source.

Brief Description of the Drawings

[Fig. 1] A graph showing the results of an anti-phage test employing a titanium oxide-coated glass plate (1.5 mg/25 $cm^2$).

[Fig. 2] A graph showing the results of an anti-phage test employing a Cu ion-containing titanium oxide-coated glass plate (8.5 mg/6.25 $cm^2$).

[Fig. 3] A graph showing the results of an anti-phage test employing a Cu ion-containing titanium oxide-coated glass plate (2 mg/6.25 $cm^2$) .

[Fig. 4] A graph showing the relationship between virus titer and light irradiation time in Examples 7 and 8 and Comparative Examples 9 to 11.

[Fig. 5] A graph showing the relationship between virus titer and light irradiation time in Example 9 and Comparative Example 12.

[Fig. 6] A graph showing the relationship between virus titer and light irradiation time in Examples 10 and 11 and Comparative Examples 9, 12, and 13.

[Fig. 7] Electrophoresis images of virus proteins obtained at different irradiation times. (A) shows the results of Comparative Example 14, and (B) shows the results of Example 12.

[Fig. 8] A graph showing the results of an anti-phage test employing a titanium oxide-coated glass plate (1.5 mg/25 $cm^2$). Modes for Carrying Out the Invention

A characteristic feature of the virus inactivation method of the present invention resides in that a virus which is in contact with a photocatalyst material is inactivated by irradiating the photocatalyst material with light from a light source. Particularly, the virus is inactivated through partial break down of the membrane protein structure of the virus. Generally, a virus has a structure in which nucleic acid is encapsulated by a protein shell called "capsid." On the outer surface of capsid, a lipid bilayer membrane containing protein (called an "envelope") is present. It is known that a virus adsorbs onto a host cell by the mediation of protein contained in the envelope, to thereby enter the cell, which triggers proliferation of the virus. Meanwhile, a photocatalyst material is known to exhibit on the surface thereof a strong oxidation or reduction property when received excitation-inducing light.

Extensive studies conducted by the inventors have revealed that the titer-decreasing rate of a virus is faster than the nucleic-acid-decreasing rate thereof on a light-received photocatalyst material. Thus, the virus inactivation mechanism employed by the photocatalyst has been elucidated as follows. That is, membrane protein present on the surface of the virus is partially damaged by a strong oxidation or reduction property provided by the photocatalyst, whereby the titer (infection ability) of the virus is reduced. In addition, the internal nucleic acid of the virus is not necessarily decomposed in the virus inactivation. When the membrane protein present on the surface of the virus has been partially damaged, the virus no longer adsorbs onto cells. In this case, the virus substantially fails its infection ability to a host cell.

The present invention has first elucidated a method for inactivating a virus through a combination of a photocatalyst material and light (energy) and the inactivation mechanism thereof. Also, the invention has first proven that a virus can be effectively inactivated through only visible light.

Nakano *et al.* have reported that the inactivation behavior of a virus on a photocatalyst is similar to that of a bacteriophage on a photocatalyst (Bulletin Photocatalyst, Vol. 29, p. 38, July, 2009).

In the virus inactivation method of the present invention, a UV-light-responsive photocatalyst material or a visible-light-responsive photocatalyst material is used as a photocatalyst material.

[UV-light-responsive photocatalyst material]

The UV-light-responsive photocatalyst material is a material which exhibits a catalytic activity induced by light including a UV ray having a wavelength of 350 to 400 nm. Examples of the photocatalyst material include titanium oxide. Titanium oxide is known to have a crystal structure type of anatase type, rutile type, or brookite type. Titanium oxide having any of these crystal structure types may be employed. These titanium oxide species may be produced through a known method such as vapor phase oxidation, the sol-gel method, or the hydrothermal method.

The UV-light-responsive photocatalyst material is preferably employed as a coating film. In this case, the aforementioned titanium oxide is mixed with a binder (e.g., silica binder), and the titanium oxide content of the formed coating film is preferably adjusted to 10 $mg/m^2$ to 10 $g/m^2$, more preferably 100 $mg/m^2$ to 3 $g/m^2$, for attaining practical photocatalytic activity. In addition to titanium oxide, a photocatalyst promoter such as a platinum-group metal (e.g., platinum, palladium, rhodium, or ruthenium) is preferably incorporated into the photocatalyt material. The photocatalyst promoter content is generally adjusted to 1 to 20 mass% (as the total content with titanium oxide), from the viewpoint of photocatalytic activity.

In the present invention, the aforementioned UV-light-responsive photocatalyst material is irradiated with light including a UV ray having a wavelength of 350 to 400 nm, whereby membrane protein of a virus which is in contact with the photocatalyst material is partially destroyed, to thereby inactivate the virus.

Examples of the light source which may be used in the invention include a black fluorescent lamp, an LED, and an organic EL, each providing light having a wavelength of 350 to 400 nm.

[Visible-light-responsive photocatalyst material]

The visible-light-responsive photocatalyst material is a material which exhibits a catalytic activity induced by light including visible light having a wavelength of 400 to 530 nm.

The visible-light-responsive photocatalyst material preferably employed in the present invention is a combination of (A-1) a copper compound and/or an iron compound or (A-2) any of platinum, palladium, rhodium, and ruthenium, or a mixture of two or more species thereof, with (B) at least one member selected from among tungsten oxide, titanium oxide, and titanium oxide having a conduction band controlled through doping. In the present invention, ingredients (A-1) and (A-2) may be referred to collectively as ingredient (A).

(Ingredient (A-1))

In the visible-light-responsive photocatalyst material, a copper compound and/or an iron compound are(is) used as ingredient (A-1). The copper compound or iron compound is preferably a compound which serves as a multi-electron oxygen-reduction catalyst for the ingredient (B) mentioned hereinbelow and can smoothly transfer electrons (e.g., a divalent copper salt or a trivalent iron salt).

Examples of the salt form of the divalent copper salt or a trivalent iron salt include halides (chloride, fluoride, bromide, and iodide), acetates, sulfates, and nitrates.

In the present invention, ingredient (A-1) may be a single species or two or more species of divalent copper salt, or a single species or two or more species of trivalent iron salt. Alternatively, one or more species of divalent copper salt and one or more species of trivalent iron salt may be used in combination.

Notably, as described hereinbelow, the copper compound or the iron compound serving as ingredient (A-1) is preferably deposited on the surface of a photocatalyst serving as ingredient (B): at least one member selected from among tungsten oxide, titanium oxide, and doped titanium oxide, from the viewpoint of the catalytic activity of the visible-light-responsive photocatalyst material.

(Ingredient (A-2))

In the visible-light-responsive photocatalyst material, any of platinum, palladium, rhodium, and ruthenium, or a mixture of two or more species thereof serving as ingredient (A-2). Ingredient (A-2) serves as a multi-electron oxygen-reduction catalyst for the ingredient (B) mentioned hereinbelow.

From the viewpoints of the performance and cost of the multi-electron reduction catalyst, ingredient (A-1) is more preferably than ingredient (A-2). Ingredient (A-2) may be additionally added to ingredient (A-1).

(Ingredient (B))

In the visible-light-responsive photocatalyst material, at least one member selected from among tungsten oxide, titanium oxide, and titanium oxide having a conduction band controlled through doping is used as a photocatalyst serving as ingredient (B).

<Tungsten oxide>

Tungsten oxide ($WO_3$) is known to absorb visible light, which generally exhibits considerably low photocatalytic activity. Recently, however, Patent Document 1 discloses that a combination of tungsten oxide and a copper compound serving as a catalyst-activity-promoter is a useful visible-light-responsive photocatalyst material. Non-Patent Document 1 discloses that tungsten oxide containing copper ions or iron ions is a useful visible-light-responsive photocatalyst material. That is, tungsten oxide, when combined with the aforementioned ingredient (A), in particular a copper compound, can serve as an effective visible-light-responsive photocatalyst material.

The copper compound and tungsten oxide may be combined through, for example, a method in which CuO powder (about 1 to about 5 mass%) is added to tungsten oxide powder, or a method in which a polar solvent solution containing a divalent copper salt (e.g., copper chloride, copper acetate, copper sulfate, or copper nitrate) is added to tungsten oxide powder, and then the mixture is dried and fired at about 500 to about 600°C, to thereby deposit copper ions on the surfaces of tungsten oxide particles. The amount of copper ion deposited on tungsten oxide may be appropriately selected in accordance with, for example, the morphology of the formed visible-light-responsive photocatalyst material, but the amount is preferably 0.001 to 0.1 mass% (as metallic Cu) with respect to tungsten oxide, more preferably 0.002 to 0.05 mass%. When the amount of copper ion is 0.001 to 0.1 mass%, an inexpensive and high-performance photocatalyst can be produced.

<Titanium oxide>

In order to attain excellent photocatalytic activity of titanium oxide serving as ingredient (B) under irradiation with visible light, preferably, titanium oxide is combined with ingredient (A), to thereby form, for example, copper-modified titanium oxide or iron-modified titanium oxide. No particular limitation is imposed on the crystal structure type of the raw material titanium oxide, and any of anatase type, rutile type, and brookite type may be employed.

More preferably, the copper-modified titanium oxide serving as an effective visible-light-responsive photocatalyst material has a crystal structure including at least brookite-type crystals. When brookite-type crystals are included, hydrous titanium oxide, titanium hydroxide, titanic acid, amorphous titanium oxide, anatase-type crystals, rutile-type crystals, etc. may be present.

The presence of brookite-type crystals may be confirmed through powder X-ray diffraction analysis employing Cu-K$\alpha$1 line. More specifically, the brookite crystal can be identified when a power X-ray diffraction line is detected at least at an interplanar spacing d (Å) of 2.90 ± 0.02 Å.

Through comparison among a peak attributed to brookite-type crystal (2.90 Å), a peak attributed to anatase-type crystal (2.38 Å), and a peak attributed to rutile-type crystal (3.25 Å), the presence of each crystal phase in titanium oxide and the relative content thereof can be roughly known. However, the relative intensities of the three peaks do not completely coincide with the relative contents of titanium oxide, and the presence of amorphous titanium oxide is ignored. Therefore, the crystal phase content is preferably determined through the Rietveld method employing an internal standard.

Specifically, the brookite-type crystal content may be determined through the Rietveld method employing 10 mass% nickel oxide serving as an internal standard. The ratio of each crystal may be determined by, for example, Rietveld analysis software in the X' Pert High Score Plus program (product of Panalytical).

The brookite-type crystal content is preferably 14 mass% to 60 mass%, more preferably 14 mass% to 40 mass%.

When the brookite-type crystal content is 14 mass% or higher, dispersion of titanium oxide sol and adsorption of copper ion species onto titanium oxide are enhanced, which is preferred. When a photocatalyst employs such titanium oxide, excellent catalytic performance can be attained. When the brookite-type crystal content is 60 mass% or lower, interaction between surface copper ion species and titanium oxide can be maintained in a favorable state without increasing the crystallite size.

The crystallite size of brookite-type crystals is preferably 12 nm or less, more preferably 5 to 12 nm. When the crystallite size is 12 nm or less, interaction between titanium oxide and copper ions is advantageously enhanced, and reactivity between photocatalyst particle surfaces and copper ions is modified, to thereby enhance sensitivity to visible light.

The crystallite size of crystals is determined through Scherrer's equation:

[F1]

$$t = \frac{0.9\,\lambda}{\sqrt{B_M{}^2 - B_S{}^2}\,\cos\theta}$$

(wherein t represents crystallite size (nm), $\lambda$ represents the wavelength of X-ray (Å), $B_M$ represents half-width of sample, $B_S$ represents half-width of reference ($SiO_2$), and $\theta$ represents diffraction angle).

The surface of the modified titanium oxide is modified with copper ion species. Examples of the copper ion species include those derived from copper(II) chloride, copper(II) acetate, copper(II) sulfate, copper(II) nitrate, copper(II) fluoride, copper(II) iodide, copper(II) bromide, etc. Among them, copper ion species derived from copper(II) chloride is preferred, in consideration of availability and productivity.

Copper ion species are formed via chemical reaction (e.g., decomposition or oxidation of the aforementioned percursor on titanium oxide or via physical transformation (e.g., deposition).

The amount of copper ion species modifying titanium oxide is preferably 0.05 to 0.3 mass% (as metallic Cu), more preferably 0.1 to 0.2 mass%.

When the modification amount is 0.05 mass% or more, the photocatalytic performance of the produced photocatalyst is excellent, whereas when the amount is 0.3 mass% or less, aggregation of copper ion species is prevented, and impairment in photocatalytic performance of the produced photocatalyst is prevented.

Conceivable reasons why the copper-modified titanium oxide exhibits photocatalytic activity even under irradiation with visible light are that direct electron transition from the valance band of brookite-type titanium oxide to copper ions occurs under irradiation with light and that the interaction between copper ion species and titanium oxide is promoted by virtue of brookite-type crystal structure, thereby attaining photocatalytic activity more excellent than that of conventional titanium oxide.

Particularly when two different crystal structure types; i.e., anatase type and rutile type, having different band gaps are present, charge separation of photo-generated electron electrons and holes is promoted, possibly enhancing photocatalytic activity. Therefore, conceivably, the presence of titanium oxide species having different band gaps promotes charge separation, whereby excellent characteristics of titanium oxide including brookite-type crystals are provided.

The copper-modified titanium oxide may be produced via, for example, a hydrolysis step of hydrolyzing a titanium compound forming titanium oxide in reaction solution, and a surface modification step of performing surface modification of titanium oxide by mixing the hydrolysis product solution with an aqueous solution containing copper ion species.

In the hydrolysis step, a titanium compound solution (e.g., an aqueous titanium chloride solution) is hydrolyzed to thereby form a titanium oxide slurry. Through tuning the conditions of the solution during hydrolysis, a crystal form of interest can be selectively produced. For example, titanium oxide particles having a brookite content of 7 to 60 mass% are produced. In addition, the crystallite size calculated from the half-width of an X-ray diffraction peak and Scherrer's equation can be adjusted to, for example, 9 to 24 nm. The crystal structure and the crystallite size of titanium oxide considerably changes the mobility of photo-generated carriers and the interaction between titanium oxide and copper ions.

Specific conditions under which the above hydrolysis is performed include (1) a hydrolysis and aging temperature of 60 to 101°C, (2) dropwise addition of aqueous titanium tetrachloride solution at a rate of 0.6 g/min to 2.1 g/min, (3) addition of hydrochloric acid in an amount of 5 to 20 mass%, and (4) any combination thereof. Through modifying these conditions, the crystal phase and crystallite size of the produced modified titanium oxide can be selected.

In the aforementioned surface modification step, the surface modification temperature is preferably 80 to 95°C, more preferably 90 to 95°C. Through adjusting the temperature to 80 to 95°C, the titanium oxide surface can be effectively modified with Cu ions.

Modification of titanium oxide with copper ion species may be carried out through methods disclosed in Non-Patent Document 1, for example, method (1) in which photocatalyst particles and copper chloride are mixed and heated in a liquid medium, followed by washing with water, or method (2) photocatalyst particles and copper chloride are mixed and heated in a liquid medium, followed by evaporation to solid. Of these, method (1) is preferred, since counter anions can be removed without heating.

The iron-modified titanium oxide serving as an effective visible-light-responsive photocatalyst material may have a crystal form of any of anatase type, rutile type, or brookite type, or a mixed form thereof. The iron-modified titanium oxide preferably contains a titanium oxide form with high crystallinity. In other words, the amount of amorphous titanium oxide or titanium hydroxide is preferably small. Such high crystallinity can be detected by a small peak half-width observed in powder X-ray diffractometry.

<Titanium oxide having a conduction band controlled through doping>

The titanium oxide having a conduction band controlled through doping, serving as ingredient (A), is titanium oxide doped with a metal ion which can positively shift the conduction band lowest potential of titanium oxide, or with a metal ion which can form an isolated (undegenerated) level on the positive side of the conduction band lowest potential of titanium oxide. Examples of such a metal ion include tungsten(VI), gallium(III), cerium(IV), germanium(IV), and barium(V). These metal ions may be used as a dopant singly or as dopants in combination or two or more species. Particularly preferred examples of the titanium oxide having a conduction band controlled through doping which may be employed in the invention include titanium oxide doped with tungsten (i.e., tungsten-doped titanium oxide (hereinafter may be referred to as "W-doped titanium oxide") and titanium oxide doped with tungsten and gallium (i.e., tungsten/gallium-co-doped titanium oxide (hereinafter may be referred to as "W/Ga-co-doped titanium oxide")).

The visible-light-responsive catalyst material of the present invention preferably contains any of these doped titanium oxide species, and a combination of a copper compound and an iron compound (ingredient (B)). Particularly, a divalent copper salt and/or a trivalent iron salt is preferably deposited on the surface of the doped titanium oxide.

As described above, in the present invention, titanium oxide is doped with tungsten as a dopant. Conceivable reasons why tungsten serves as a suitable dopant are as follows.

One reason is that the lowest potential of the conduction band formed in titanium oxide is appropriately shifted to the positive side through doping with tungsten. The shift can be estimated by calculating the density of state of a semiconductor through a known technique disclosed in a published document (K. Obata et al., Chemical Physics, vol. 339, p. 124-132, 2007).

Another reason is that tungsten(VI) has an ionic radius of 0.58 Å, which is approximately equal to that of titanium(IV) (0.61 Å), whereby tungsten(VI) is readily substituted by titanium(IV) in the crystal.

Other than tungsten, there are some metals which satisfy the above conditions to provide the above effects. However, tungsten is a particularly suitable dopant for a certain reason which has not been clearly elucidated. Conceivable reasons are as follows. One reason is that electron transfer is smoothly attained to the divalent copper salt or trivalent iron salt which serves as a multi-electron oxygen reduction catalyst and which has been deposited on the surface of titanium oxide. Another reason is that tungsten easily occupies the titanium sites.

In the present invention, no particular limitation is imposed on the morphology of titanium oxide to be doped, and titanium oxide microparticles and titanium oxide thin film may be employed. Since a photocatalyst having a large specific surface area is advantageous for photocatalytic reaction, titanium oxide microparticles are particularly preferred. Also, no particular limitation is imposed on the crystal structure type of titanium oxide, and rutile type, anatase type, brookite type, etc. may be employed.

In the case where titanium oxide includes rutile-type crystals as a main component, the rutile-type content is preferably 50% or more, more preferably 65% or more. In the case where titanium oxide includes anatase-type or brookite-type crystals as a main component, the above range is also preferred.

The ratio of each crystal phase may be derived from a specific X-ray diffraction peak. When titanium oxide includes rutile-type crystals as a main component, the ratio of the intensity of the peak attributed to rutile structure type to the sum of the intensities of peaks attributed to all titanium oxide crystal structure types is calculated.

In the present invention, the amount of doping with tungsten is preferably such that the ratio by mole of tungsten to titanium (W:Ti by mole) falls within a range of 0.01:1 to 0.1:1. When the W:Ti mole ratio is 0.01:1 or more, visible light absorption can be sufficiently increased through doping with tungsten. When the W:Ti mole ratio is 0.1:1 or less, defects of titanium oxide crystals are reduced, and recombination of photogenerated electrons and holes is suppressed, while visible light absorption is increased. In this case, the efficiency of the photocatalyst can be maximized. As is clear from the above description, the optimum W:Ti mole ratio is determined in consideration of the balance between reduction of defects of titanium oxide crystals and increase in visible light absorption through doping with tungsten. Thus, the W:Ti mole ratio preferably falls within a range of 0.01:1 to 0.05:1, more preferably 0.02:1 to 0.04:1.

The dopant employed in the present invention may be tungsten singly (W-doped titanium oxide), but co-doping with tungsten and gallium is preferred.
In the case of W-doped titanium oxide, tungsten(VI) ions replace titanium(IV) ion sites, whereby positive charges are excessively present. Thus, in order to adjust the positive charge-electron balance, tungsten(V) or tungsten(IV), or oxygen defect, is thought to generate. Since these structural defects fall outside the anticipated band structure, light absorption might become insufficient, or recombination of photoexcited electrons and holes might occur, possibly resulting in lowering of photocatalyst activity.
Then, the presence of gallium(III) ions may appropriately maintain the balance. Also, the ionic radius gallium(III) is 0.62 Å, which is approximately equal to that of titanium(IV) (0.61 Å). Therefore, co-doping with gallium and tungsten is preferred. In consideration of the aforementioned charge balance, the amount of doping with gallium is ideally such that the ratio by mole of tungsten to gallium (W:Ga mole ratio) is 1:2. Thus, the W:Ga mole ratio is preferably equal to 1:2, preferably at least falling within a range of 1:1.5 to 1:2.5, more preferably 1:1.7 to 1:2.3, yet more preferably 1:1.8 to 1:2.2.

As described above, the divalent copper salt and trivalent iron salt deposited on the surface of titanium oxide are thought to serve as a multi-electron oxygen reduction catalyst for realizing smooth electron transfer, and as a result, oxidation-decomposition activity under irradiation with visible light is thought to be enhanced. The amount of each of the divalent copper salt and the trivalent iron salt deposited on the titanium oxide surface is preferably 0.0001 to 1 mass% of the photocatalyst material, more preferably 0.01 to 0.3 mass%.

From the viewpoint of imparting anti-bacterial property to the photocatalyst material in the dark, a divalent copper salt is preferred, whereas from the viewpoint of material safety (non-toxicity), a trivalent iron salt is preferred.
The divalent copper salt and trivalent iron salt include a precursor thereof and various species formed by oxidation or decomposition during a step of depositing the salts.

The visible-light-responsive photocatalyst material of the present invention preferably has a particle size of 0.005 to 1.0 $\mu$m in consideration of its activity and handling property, more preferably 0.01 to 0.3 $\mu$m. The particle size may be adjusted through classification by means of a sieve or a similar technique.

The visible-light-responsive photocatalyst material of the present invention may be produced through a method including a doping step of producing W-doped titanium oxide or W/Ga-co-doped titanium oxide and a metal salt deposition step for depositing a divalent copper salt and/or a trivalent iron salt on doped titanium oxide.

In the doping step, no particular limitation is imposed on the method of producing W-doped titanium oxide or W/Ga-co-doped titanium oxide, but the following four methods are effective.
Specifically, the methods are:

  (1) a method of producing W-doped titanium oxide or W/Ga-co-doped titanium oxide through the so-called sol-gen process;
  (2) a method in which a solution containing a tetravalent titanium salt is added to a dopant solution which has been heated to a predetermined temperature, to thereby form W-doped titanium oxide or W/Ga-co-doped titanium oxide;
  (3) a so-called vapor-phase synthesis method; i.e., mixing a gas containing vapor of a volatile titanium compound and vapor of a volatile tungsten compound, or a gas containing vapor of a volatile titanium compound, vapor of a volatile tungsten compound, and vapor of a volatile gallium compound, with a gas containing oxidizing gas, to thereby form W-doped titanium oxide or W/Ga-co-doped titanium oxide; and
  (4) a method in which a hexavalent tungsten salt, or a hexavalent tungsten salt and a trivalent gallium salt are deposited on the surface of titanium oxide powder, followed by firing at about 800 to about 1000°C, to thereby form W-doped titanium oxide or W/Ga-co-doped titanium oxide.

In the metal salt deposition step, a divalent copper salt and/or a trivalent iron salt are(is) deposited on the surface of the W-doped titanium oxide or W/Ga-co-doped titanium oxide produced through the aforementioned method.

The divalent copper salt and/or trivalent iron salt is preferably deposited on the surface of the metal-doped titanium oxide in the form of a very thin layer (highly dispersed microparticles). The reason for this has not been clearly elucidated, but one estimation is that a large mass of elemental copper or elemental iron is not suited for forming a structure suitable for receiving electrons excited to the valance band and for multi-electron reduction of oxygen. Therefore, the divalent copper salt and/or trivalent iron salt is preferably deposited on the surface of the metal-doped titanium oxide in the form of a very thin layer. Such a material is suitably produced through the following method.

Specifically, the method includes bringing W-doped titanium oxide and/or W/Ga-co-doped titanium oxide into contact with an aqueous solution of a divalent copper salt and/or trivalent iron salt and heating the mixture at about 85 to about 100°C (preferably 90 to 98°C). Through the procedure, only copper ions and iron ions are bonded to the surface of titanium oxide in water at 85 to 100°C. The solid is recovered through filtration or centrifugation and sufficiently washed. The inventors have found that counter ions to copper ions and iron ions are preferably removed sufficiently in the water washing step so that the activity of the produced visible-light-responsive photocatalyst increases. Therefore, the divalent copper salt and/or trivalent iron salt deposited on the surface of titanium oxide is supposed to assume the form of corresponding cations with hydroxide ions as counter ions.

The thus-produced visible-light-responsive photocatalyst material is a photocatalyst material containing at least one member selected from among, for example, a mixture of tungsten oxide powder and CuO powder, copper-ion-deposited tungsten oxide, copper-modified titanium oxide, copper-modified titanium oxide containing brookite-type crystals, iron-modified titanium oxide, divalent copper salt-deposited and/or trivalent iron salt-deposited W-doped titanium oxide, and divalent copper salt-deposited and/or trivalent iron salt-deposited W/Ga-co-doped titanium oxide.

The visible-light-responsive photocatalyst material is preferably employed in the form of coating film. In this case, the visible-light-responsive photocatalyst material content of the coating film is preferably adjusted to 100 mg/m$^2$ to 20 g/m$^2$, more preferably 500 mg/m$^2$ to 15 g/m$^2$, for attaining practical photocatalytic activity.

In the present invention, through irradiation of such a visible-light-responsive photocatalyst material with light including visible light having a wavelength of 400 to 530 nm, any membrane protein of envelope, envelope protein, and capsid-forming protein of the virus which is in contact with the photocatalyst material is partially destroyed, to thereby inactivate the virus.

The light source which may employed in the invention provides light having a wavelength of 400 to 530 nm and is sunlight, a fluorescent lamp, an LED, an organic EL, etc.

(Virus)

A virus is a small penetrable pathogen which is smaller than a bacterium, which has DNA genomes or RNA genomes, and which can replicate only inside the host cells. Examples of the type of virus include double-stranded DNA virus, single-stranded DNA virus, double-stranded RNA virus, single-stranded RNA virus, and viruses belonging to the retrovirus family. Viruses are classified into those having an envelope (lipid bilayer) and those not having it.

The method of the present invention can be generally applied to any virus regardless of the presence or absence of an envelope and is particularly effective when applied to influenza viruses and bacteriophages. The term "bacteriophage" refers to a virus which infects a bacterium as a host. Examples of known influenza viruses include avian influenza viruses and swine influenza viruses.

[Article provided with an anti-viral property]

The present invention also provides an article comprising a visible-light-responsive photocatalyst material deposited on a surface thereof and exhibiting an anti-viral property.

The visible-light-responsive photocatalyst material to be deposited on the surface of the article is preferably a combination of (A) a copper compound and/or an iron compound, with (B) at least one member selected from among tungsten oxide, titanium oxide, and titanium oxide having a conduction band controlled through doping. Details of such visible-light-responsive photocatalyst materials are described above.

When the visible-light-responsive photocatalyst material of the article provided with an anti-viral property is irradiated with light including visible light having a wavelength of 400 to 530 nm, membrane protein of a virus present in the vicinity of the light irradiation area is partially destroyed, whereby the virus can be effectively inactivated.

The article provided with an anti-viral property of the present invention finds a variety of uses. Examples of the article for intentionally remove viruses include a filter for an air-purifier, as well as a cover or a light-reflecting back panel of an illumination device. Examples of the article employed for routinely reducing viruses include a wall panel, ceiling, floor, step, handrail, door, papered sliding door (*fusuma* or *shoji*), door knob, and handle of any living space such as a dwelling, office, factory, hospital, toilet, bath room, kitchen, washroom, or corridor. Examples also include a ceiling, wall panel, floor, seat, window pane, window frame, and hand rail of any transportation vehicle such as an automobile, train, aircraft, or bus.

Examples

The present invention will next be described in more detail by way of Examples, which should not be construed as limiting the invention thereto.

Notably, "Qβ phage" and "T4 phage" employed in the following Examples are species of bacteriophages. A bacteriophage is a virus which infects cells. Qβ phage is an RNA phage having a length of about 25 nm and an icosahedral structure, while T4 phage is a DNA phage having a length of about 200 nm with a long (icosahedral) head and a shrinkable tail.

Example 1

(Production of titanium oxide-coated glass plate)

An aqueous slurry containing titanium oxide microparticles (FP-6, product of Showa Titanium) for photocatalyst use in an amount of 10 mass% was prepared. The titanium oxide microparticles were dispersed through ultrasonication for at least 5 minutes by means of an ultrasonic washing apparatus. The thus-prepared aqueous slurry was applied onto a glass plate (5 cm × 5 cm × 1 mm (thickness)), while the glass plated was fixed on a spin-coater, and the spin-coater was rotated. The glass plate was placed in a thermostat drier at 120°C and dried for at least one hour, to thereby prepare a titanium oxide-coated glass plate. The amount of titanium oxide on the surface of the glass plate was 1.5 mg/25 cm$^2$ (=600 mg/m$^2$). Ten plates were prepared in total.

(Anti-phage performance test)

Filter paper was placed on the inner surface of a deep Petri dish, and a small amount of sterilized water was added thereto. A glass spacer (height: about 5 mm) was placed on the filter paper, and the above-prepared titanium oxide-coated glass plate was placed on the spacer. A solution (about 100 μL) of Qβ phage which had been purified and whose concentration was known was sprayed onto the glass plate. The Petri dish was closed with a glass plate, to thereby prepare an assay set. A plurality of the assay sets were provided and allowed to stand in the dark at room temperature. The number of assay sets was the number of the phage count procedure. The assay sets were placed under a 20W black fluorescent lamp (FL20S·BLB, product of Toshiba Lighting & Technology Corp.) at a UV ray intensity of 1 mW/cm$^2$ (determined by means of a photo-power meter for photocatalyst, C9536-01+H9958, product of Hamamatsu Photonics K. K.), whereby the assay sets were irradiated with light. After irradiation for a predetermined period of time, the phage concentration of each sample was determined.

(Phage concentration determination)

The glass plate which had undergone light exposure and whose phage concentration was to be determined was immersed in a recovery liquid (PBS + Tween 20) (10 mL) and shaken in the liquid for 10 minutes by means of a shaking apparatus. The thus-recovered liquid containing a phage was appropriately diluted, and each product was mixed with separately cultured *E. coli.* solution (OD$_{600}$>1.0, 1 × 10$^8$ CFU/mL). The mixture was stirred and then allowed to stand in a thermostat container at 37°C for 10 minutes. The thus-obtained liquid was inoculated to an agar medium, and cultivation was performed at 37°C for 15 hours. The number of plaques of the phage was visually counted. The phage concentration was determined through multiplication of the plaque number and the dilution factor of the phage recovery liquid.

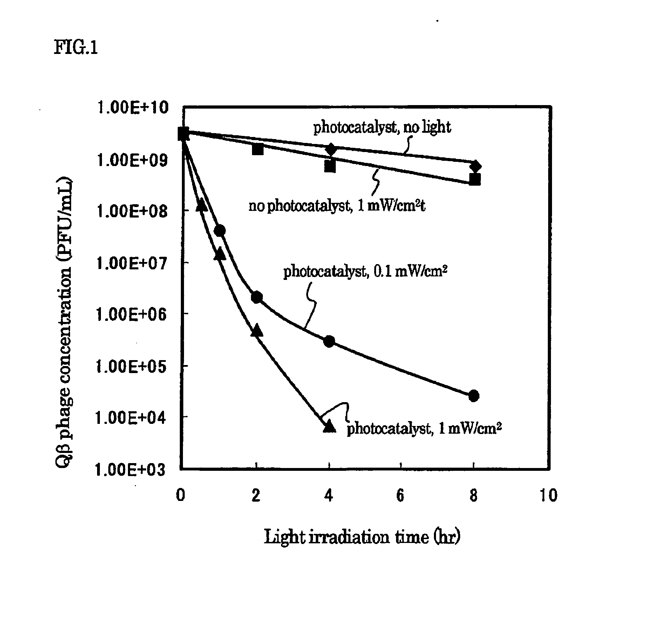

The thus-obtained time-dependent profile of Qβ phage concentration is shown in Fig. 1, indicated with "photocatalyst, 1 mW/cm$^2$."

In the experiment, PBS refers to phosphated physiological saline (product of Wako Pure Chemical Industries, Ltd.), and Tween 20 refers to polyoxyethylene(20) sorbitan monolaurate (product of Wako Pure Chemical Industries, Ltd.).

Example 2

The procedure of Example 1 was repeated, except that the UV ray intensity was adjusted to 0.1 mW/cm$^2$ in "Anti-phage performance test." The results are shown in Fig. 1, indicated with "photocatalyst, 0.1 mW/cm$^2$."

Comparative Example 1

The procedure of Example 1 was repeated, except that the sample was not irradiated with light but was placed in the dark in "Anti-phage performance test." The results are shown in Fig. 1, indicated with "photocatalyst, no light."

Comparative Example 2

The procedure of Example 1 was repeated, except that the titanium oxide was not applied onto the glass plate in "Production of titanium oxide-coated glass plate." The results are shown in Fig. 1, indicated with "no photocatalyst, 1 mW / cm$^2$ . "

As is clear from Fig. 1, Qβ phage was inactivated only in the presence of the photocatalyst and under irradiation with light.

Example 3

(Preparation of visible-light-responsive photocatalyst "copper-ion-deposited tungsten oxide")

WO$_3$ powder (mean particle size: 250 nm, product of Kojyundo Chemical Laboratory Co., Ltd.) was passed through a filter, to thereby remove particles having a particle size of 1 μm or more. The thus-treated powder was fired at 650°C for 3 hours (preliminary treatment), to thereby yield tungsten trioxide microparticles. The tungsten trioxide microparticles were suspended in distilled water (10 mass%: WO$_3$ vs. H$_2$O) , and CuCl$_2$ · 2H$_2$O (product of Wako Pure Chemical Industries, Ltd.) was added thereto in an amount of 0.1 mass% (Cu(II) vs. WO$_3$). The mixture was heated at 90°C under stirring and maintained at 90°C for one hour. Subsequently, the thus-formed suspension was filtered under suction, and the residue was washed with distilled water and dried by heating at 110°C, to thereby yield divalent-copper-salt-deposited tungsten trioxide microparticles. Test samples were obtained therefrom.

The amount of Cu(II) deposited in the divalent-copper-salt-deposited tungsten trioxide microparticles, determined through in inductively coupled plasma atomic emission spectrometry (ICP-AES, P-4010, HITACHI) and polarized Zeeman atomic absorption spectrometry (polarized Zeeman AAS, Z-2000, HITACHI), was found to be 0.0050 mass% (Cu(II) vs. WO$_3$: 5 mass% of starting material).

(Production of copper-ion-deposited tungsten oxide-coated glass plate)

An aqueous slurry containing the above-produced copper-ion-deposited tungsten oxide in an amount of 5 mass% was prepared. The copper-ion-deposited tungsten oxide microparticles were dispersed through ultrasonication for 30 minutes by means of an ultrasonic washing apparatus, to thereby disperse the microparticles. The dispersion was added dropwise to the entire surface of a glass plate (2.5 cm x 2.5 cm $\times$ 1 mm (thickness)) so as not to overflow. The glass plate was placed in a thermostat drier at 120°C and dried for one hour, to thereby prepare a copper-ion-deposited tungsten oxide-coated glass plate. The amount of copper-ion-deposited tungsten oxide on the surface of the glass plate was 8.5 mg/6.25 cm$^2$ (=13.6 g/m$^2$). Ten plates were prepared in total.

(Anti-phage performance test)

The above-produced copper-ion-deposited tungsten oxide-coated glass plate was used as a sample. The light source employed was a 15W daylight fluorescent lamp (full-white fluorescent lamp, FL15N, product of Panasonic Co.) to which a UV-cutting filter (KU-1000100, product of King Works Co., Ltd.) was attached. The assay set (sample) was placed at a site where the illuminance was 800 lx (measured by means of an illuminance meter: TOPCON IM-5). Other than the above procedure, the same operations as employed in Example 1 were also conducted.

(Phage concentration determination)

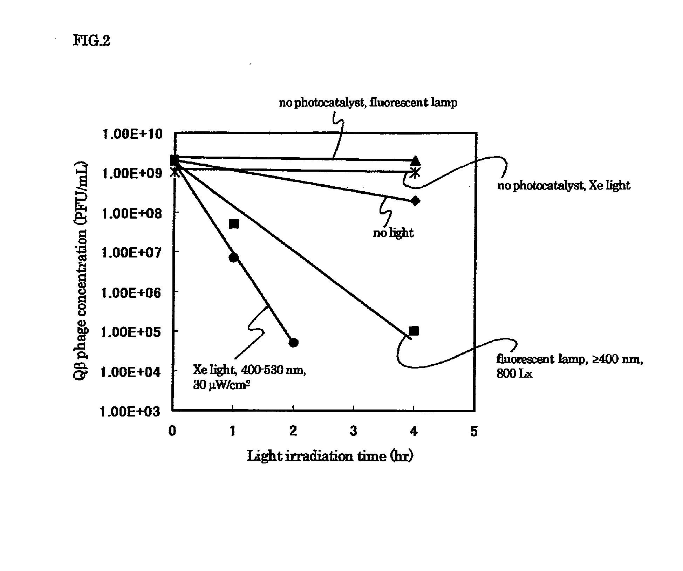

The procedure of Example 1 was repeated. The results are shown in Fig. 2, indicated with "fluorescent lamp, ≥400 nm, 800 Lx"

Example 4

The light source employed in Example 4 (Anti-phage performance test) was a xenon lamp equipped with glass filters (L-42, B-47, and C-40C, product of AGC Techno Glass Co., Ltd), to thereby limit the irradiation wavelength to 400 to 530 nm. The irradiation intensity was controlled to 30 $\mu$W/cm$^2$ (by measuring the intensity of incident light at a wavelength of interest by means of a spectroradiometer USR-45, product of Ushio Inc.). Other than the above procedure, the same operations as employed in Example 3 were also conducted. The results are shown in Fig. 2, indicated with "Xe light, 400-530 nm, 30 $\mu$W/cm$^2$."

Comparative Example 3

The procedure of Example 3 was repeated, except that the sample was not irradiated with light in "Anti-phage performance test." The results are shown in Fig. 2, indicated with "no light."

Comparative Example 4

The procedure of Example 3 was repeated, except that the photocatalyst was not applied onto the glass plate in "Production of copper-ion-deposited tungsten oxide-coated glass plate." The results are shown in Fig. 2, indicated with "no photocatalyst, only fluorescent lamp."

Comparative Example 5

The procedure of Example 4 was repeated, except that the photocatalyst was not applied onto the glass plate in "Production of copper-ion-deposited tungsten oxide-coated glass plate." The results are shown in Fig. 2, indicated with "no photocatalyst, only Xe light."
As is clear from Fig. 2, the phage was inactivated through irradiation with only visible light. In addition, the phage was inactivated only in the presence of the visible-light-responsive photocatalyst material and under irradiation with light.

Example 5

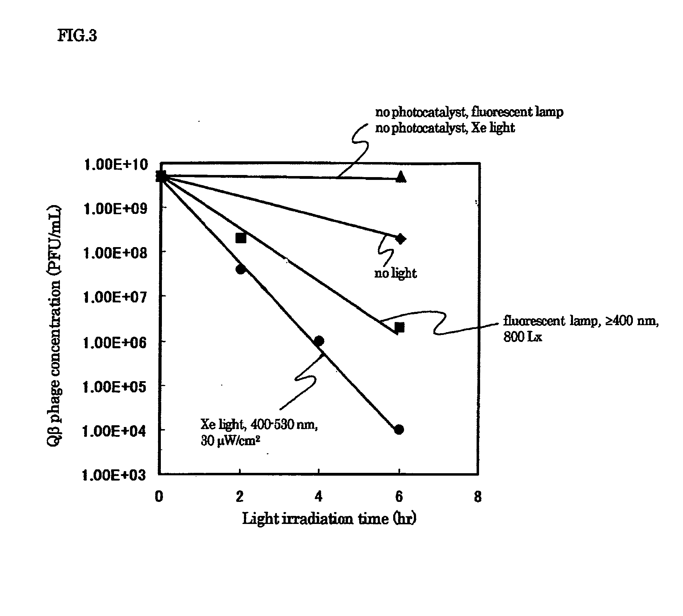

The procedure of Example 3 was repeated, except that the amount of copper-ion-deposited tungsten oxide on the surface of the glass plate was 2 mg/6.25 cm$^2$ (=3.2 g/m$^2$) in "Production of copper-ion-deposited tungsten oxide-coated glass plate." The results are shown in Fig. 3, indicated with "fluorescent lamp, ≥400 nm, 800 Lx."

Example 6

The procedure of Example 4 was repeated, except that the amount of copper-ion-deposited tungsten oxide on the surface of the glass plate was 2 mg/6.25 cm$^2$ (=3.2 g/m$^2$) "Production of copper-ion-deposited tungsten oxide-coated glass plate." The results are shown in Fig. 3, indicated with "Xe light, 400-530 nm, 30 $\mu$W/cm$^2$."

Comparative Example 6

The procedure of Example 5 was repeated, except that the sample was not irradiated with light in "Anti-phage performance test." The results are shown in Fig. 3, indicated with "no light."

Comparative Example 7

The procedure of Example 5 was repeated, except that the photocatalyst was not applied onto the glass plate "Production of copper-ion-deposited tungsten oxide-coated glass plate." The results are shown in Fig. 3, indicated with "no photocatalyst, only fluorescent lamp."

Comparative Example 8

The procedure of Example 6 was repeated, except that the photocatalyst was not applied onto the glass plate in "Production of copper-ion-deposited tungsten oxide-coated glass plate." The results are shown in Fig. 3, indicated with "no photocatalyst, only Xe light." The results were completely the same as those of Comparative Example 7.

As is clear form Fig. 3, even when the amount of the applied visible-light-responsive photocatalyst was small, anti-phage was attained.

Example 7

(Production of copper-ion-deposited tungsten oxide-coated glass plate)

The "copper-ion-deposited tungsten oxide" microparticles produced through the method described in Example 3 were pulverized by means of a mortar, and the powder was added to distilled water. The mixture were dispersed through ultrasonication for 30 minutes by means of an ultrasonic washing apparatus, to thereby prepare an aqueous slurry containing copper-ion-deposited tungsten oxide in an amount of 10 mass%.

Then, tetraethoxysilane (product of Wako Pure Chemical Industries, Ltd.) (5 parts by mass), ion-exchange water (0.8 parts by mass), 0.1-mol/L aqueous HCl (0.07 parts by mass), and ethanol (94.13 parts by mass) were mixed together in a reaction container under stirring for 16 hours, to thereby produce partial hydrolyzation-polycondensate of tetraethoxysilane.

The partial hydrolyzation-polycondensate of tetraethoxysilane (100 parts by mass) was mixed with the aqueous slurry containing copper-ion-deposited tungsten oxide (100 parts by mass) under stirring for one hour, to thereby produce a coating agent containing copper-ion-deposited tungsten oxide.

The coating agent was applied onto a clean, square glass plate (50 mm $\times$ 50 mm) by means of a spin-coater, and the coating film was dried and cured by heating at 100°C for 30 minutes, to thereby produce a copper-ion-deposited tungsten oxide-coated glass plate serving as a test sample.

(Preparation of influenza virus)

Influenza virus A/PR/8/34 (H1N1) was employed. The virus solution was inoculated to embryonated eggs (12 days old), to thereby infect the eggs with the virus, and the eggs were cultivated at 35.5°C for two days. Thereafter, the eggs were allowed to stand overnight at 4°C, and chorioallantoic liquid was collected from the eggs. The thus-collected chorioallantoic liquid was subjected to microfiltration (for removing egg-origin miscellaneous matter) and ultrafiltration (for removing impurities and concentrating virus), to thereby yield a virus concentrate. The virus concentrate was subjected to sucrose-density-gradient centrifugation (5 to 50% sucrose linear gradient, 141,000xg, for 3 hours), to thereby obtain a high-purity purified virus solution. In carrying out of the test, BSA (bovine serum albumin) was added to the virus solution as a stabilizer for stabilizing the virus.

(Anti-viral performance test)

The above-produced copper-ion-deposited tungsten oxide-coated glass plate was used as a sample. The light source employed was a 20W white fluorescent lamp (FL20S·W, product of Toshiba Lighting & Technology Corp.) to which a UV-cutting filter (N113, product of Nitto Jushi Kogyo Co., Ltd.) was attached. The assay set (sample) was placed at a site where the illuminance was 3,000 lx (measured by means of an illuminance meter: TOPCON IM-5). The above-prepared influenza virus solution was used instead of Qβ phage solution. Other than the above procedure, the same operations as employed in Example 1 (Anti-phage performance test) were also conducted.

(Virus titer measurement)

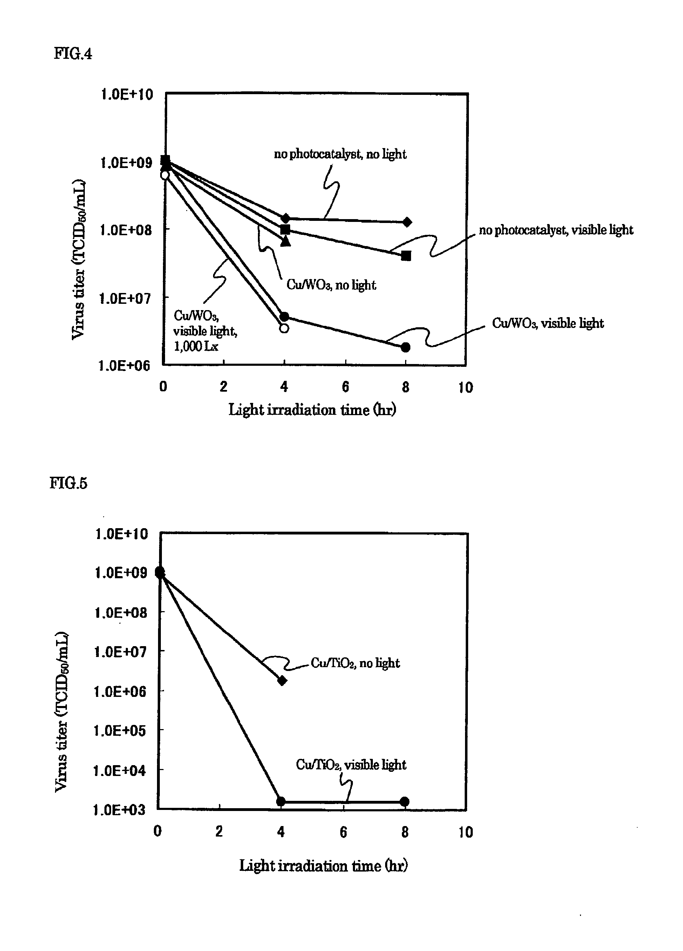

The virus-inoculated sample which had undergone light exposure was immersed in a recovery liquid (PBS + 1% BSA) (10 mL) and shaken at 100 rpm in the liquid for 10 minutes by means of a shaking apparatus, whereby the virus present on the sample was recovered. The thus-recovered influenza virus was diluted via 10-fold serial dilution to $10^{-9}$. Cultured MDCK cells (dog kidney-origin established cells) were infected with each virus solution and cultured at 37°C at a $CO_2$ concentration of 5% for 5 days. After completion of culturing, the change in cell morphology (cytopathogenic effect) was observed. The amount of virus infected 50% cultured cells was calculated through the Reed-Muench method, whereby the virus titer ($TCID_{50}$/mL) was calculated. Virus titer was measured at several points in irradiation time. Fig. 4 shows the results, indicated with "Cu/$WO_3$, visible light."

Example 8

The procedure of Example 7 was repeated, except that the assay set (sample) was placed at a site where the illuminance was 1,000 lx in "Anti-viral performance test." Fig. 4 shows the results, indicated with "Cu/$WO_3$, visible light 1,000 Lx."

Comparative Example 9

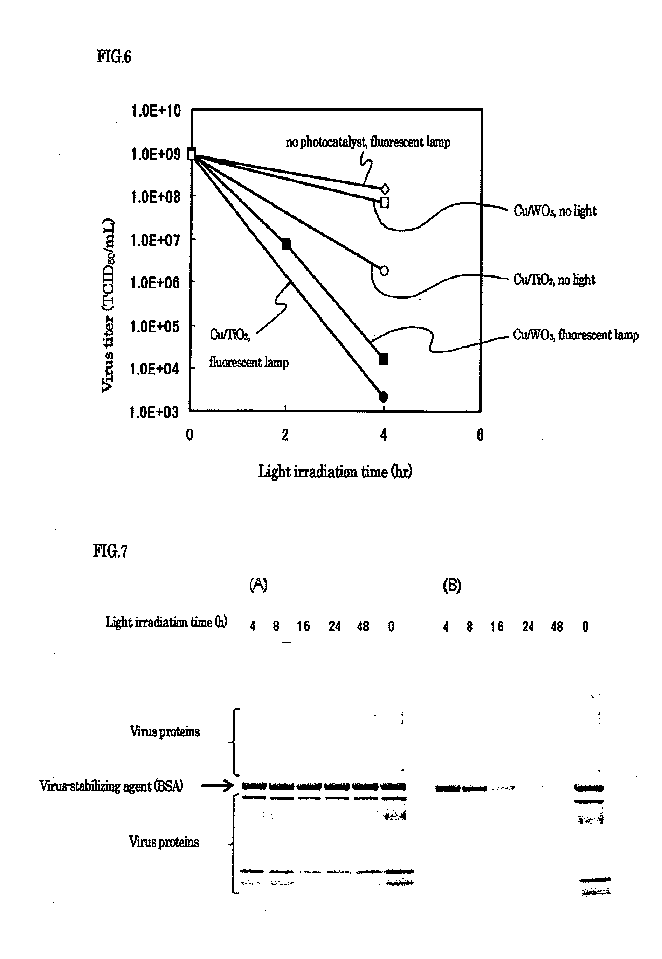

The procedure of Example 7 was repeated, except that the sample was not irradiated with light and placed in the dark in (Anti-viral performance test). The results are shown in Figs. 4 and 6, indicated with "Cu/WO$_3$, no light."

Comparative Example 10

The procedure of Example 7 was repeated, except that a non-coated glass plate was used as a sample instead of the copper-ion-deposited tungsten oxide-coated glass plate in "Anti-viral performance test." The results are shown in Fig. 4, indicated with "no photocatalyst, visible light."

Comparative Example 11

The procedure of Example 7 was repeated, except that a non-coated glass plate was used as a sample instead of the copper-ion-deposited tungsten oxide-coated glass plate, and that the sample was not irradiated with light and placed in the dark, in "Anti-viral performance test." The results are shown in Fig. 4, indicated with "no photocatalyst, no light."

As is clear from Fig. 4, the virus titer considerably decreases only when the copper-ion-modified tungsten oxide was present, and the samples were irradiated with light. Since the light with which the samples were irradiated passed through the UV-cutting filter (N113), light having a wavelength of 400 nm or less was cut off. Thus, the effect was found to be attained only by visible light.

Example 9

(Production of copper-ion-modified titanium oxide-coated glass plate)

Cu(NO$_3$)$_2$·3H$_2$O (product of Wako Pure Chemical Industries, Ltd.) was added to brookite-type titanium oxide sol (NTB-1 (registered trademark), product of Showa Titanium, solid content: 15 mass%, brookite crystal phase content of solid: 55 mass%, mean crystallite size: 10 nm) so that the amount of the copper salt was adjusted to 0.1 mass% with respect to brookite-type titanium oxide. The mixture was heated to 90°C and maintained at 90°C for one hour under stirring. Subsequently, the suspension was centrifuged, to thereby recover the solid. The thus-recovered solid was washed with distilled water and subjected to centrifugation again. This procedure was carried out three times in total. Thereafter, distilled water was added to the solid so as to adjust the solid content to 10 mass%, and the mixture was ultrasonicated by means of a ultrasonic washing apparatus, to thereby form a dispersion, whereby a copper-ion-modified titanium oxide slurry (copper ion amount: 0.1 mass% vs. titanium oxide) having a solid content of 10 mass% was yielded.

Then, tetraethoxysilane (product of Wako Pure Chemical Industries, Ltd.) (5 parts by mass), ion-exchange water (0.8 parts by mass), 0.1-mol/L aqueous HCl (0.07 parts by mass), and ethanol (94.13 parts by mass) were mixed together in a reaction container under stirring for 16 hours, to thereby produce partial hydrolyzation-polycondensate of tetraethoxysilane.

The partial hydrolyzation-polycondensate of tetraethoxysilane (100 parts by mass) was mixed with the aforemntioned dispersion containing divalent copper-salt-deposited rutile-type titanium dioxide microparticles (60 parts by mass) under stirring for one hour, to thereby produce a coating agent containing copper-ion-modified titanium oxide (copper ion content: 0.1 mass%).

The copper-ion-modified titanium oxide coating agent was applied onto a clean, square glass plate (50 mm × 50 mm) through spin coating, and the coating film was dried and cured by heating at 100°C for 30 minutes, to thereby produce a copper-ion-modified titanium oxide-coated glass plate serving as a test sample.

(Anti-viral performance test)

The procedure of Example 7 was repeated, except that the above-produced copper-ion-modified titanium oxide-coated glass plate was used as a sample.

(Virus titer measurement)

The measurement was performed in a manner similar to that employed in Example 7. Fig. 5 shows the results, indicated with "Cu/TiO$_2$, visible light."

Comparative Example 12

The procedure of Example 9 was repeated, except that the sample was not irradiated with light but was placed in the dark in "Anti-viral performance test." The results are shown in Figs. 5 and 6, indicated with "Cu/TiO$_2$, no light."

As is clear from Fig. 5, the copper-ion-modified titanium oxide-coated glass plate was found to reduce virus titer even though no light was applied. Although the mechanism has not been clearly elucidated, one conceivable reason is that copper-ion-modified titanium oxide exhibits a certain anti-viral property which differs from the effect of the present invention. However, as is clear from Fig. 5, the anti-viral performance as drastically enhanced by irradiating the sample with visible light, indicating that the anti-viral mechanism by the action of photocatalyst according to the present invention was realized.

Example 10

In "Anti-viral performance test," a 20W white fluorescent lamp (FL20S·W, product of Toshiba Lighting & Technology Corp.) to which no UV-cutting filter was attached was employed as a light source so that the sample was directly irradiated with the light from the fluorescent lamp. The assay set (sample) was placed at a site where the illuminance was 1,000 lx (measured by means of an illuminance meter: TOPCON IM-5). Other than the above procedure, the same operations as employed in Example 7 were also conducted. Fig. 6 shows the results, indicated with "Cu/WO$_3$, fluorescent lamp."

Comparative Example 13

The procedure of Example 10 was repeated, except that a non-coated glass plate was used instead of the copper-ion-deposited tungsten oxide-coated glass plate in "Anti-viral performance test." Fig. 6 shows the results, indicated with "no photocatalyst, fluorescent lamp."

Example 11

In "Anti-viral performance test," a 20W white fluorescent lamp (FL20S·W, product of Toshiba Lighting & Technology Corp.) to which no UV-cutting filter was attached was employed as a light source so that the sample was directly irradiated with the light from the fluorescent lamp. The assay set (sample) was placed at a site where the illuminance was 1,000 lx (measured by means of an illuminance meter: TOPCON IM-5). Other than the above procedure, the same operations as employed in Example 9 were also conducted. Fig. 6 shows the results, indicated with "Cu/TiO$_2$, fluorescent lamp."

As is clear from Fig. 6, the anti-viral action was attained by the photocatalyst, even when light from a fluorescent lamp to which no UV-cutting filter was attached and which provided weak UV light was employed as a light source.

Example 12

(Production of titanium oxide-coated glass)

Tetraethoxysilane (product of Wako Pure Chemical Industries, Ltd.) (5 parts by mass), ion-exchange water (0.8 parts by mass), 0.1-mol/L aqueous HCl (0.07 parts by mass), and ethanol (94.13 parts by mass) were mixed together in a reaction container under stirring for 16 hours, to thereby produce partial hydrolyzation-polycondensate of tetraethoxysilane.

The partial hydrolyzation-polycondensate of tetraethoxysilane (100 parts by mass) was mixed with an aqueous slurry containing titanium oxide micrtoparticles for photocatalyst in an amount of 10 mass% (100 parts by mass) under stirring for one hour, to thereby produce a coating agent containing titanium oxide.

The titanium oxide coating agent was applied onto a clean, square glass plate (50 mm × 50 mm) through spin-coating, and the coating film was dried and cured by heating at 100°C for 30 minutes, to thereby produce a titanium oxide-coated glass plate serving as a test sample.

(Test of photocatalytic reaction of influenza virus)

The photocatalyst reaction test was pereformed in accordance with the anti-bacterial performance test for photocatalyst products described in JIS R 1702. Specifically, filter paper was placed on the inner surface of a deep Petri dish, and a small amount of sterilized water was added thereto. A glass spacer (height: about 5 mm) was placed on the filter paper, and the above-prepared titanium oxide-coated glass plate was placed on the spacer. A solution (100 μL) of an influenza virus which had been highly purified was inoculated to the glass plate. The Petri dish was closed with a glass plate, to thereby prepare an assay set. The same six assay sets (corresponding reaction times: 0, 4, 8, 16, 24, and 48 hours) were provided and allowed to stand in the dark at room temperature. The assay sets were placed under a 20W black fluorescent lamp (FL20S·BLB, product of Toshiba Lighting & Technology Corp.) at a UV ray intensity of 0.1 mW/cm$^2$ (determined by means of a photo-power meter for photocatalyst, C9536-01+H9958, product of Hamamatsu Photonics K. K.), whereby the assay sets were irradiated with light. After irradiation for a predetermined period of time, the liquid containing the virus was recovered, and proteins were extracted therefrom.

(Analysis of virus proteins)

The extracted virus proteins were fractionated through SDS-PAGE (electrophoresis conditions: 20 mA, 80 minutes). The resultant gel was stained by SYPRO Ruby protein gel stain (Invitrogen), imaged by Image-Quant 4010 (GE Healthcare), and analyzed by Image-Quant TL (GE Healthcare). The results are shown in Fig. 7(B).

Comparative Example 14

The procedure of the above Example was repeated, except that the glass plate was not coated with the photocatalyst and was not irradiated with light in "Test of photocatalytic reaction of influenza virus." The results are shown in Fig. 7(A).

As is clear from Fig. 7(A) (electrophoresis pattern of proteins), virus proteins were identified on the virus-added glass plate after irradiation of the plate with light. In contrast, as is clear from Fig. 7(B) (electrophoresis pattern of proteins), reduction of virus proteins was identified on the virus-added and titanium oxide-coated glass plate after irradiation of the plate with light. Furthermore, 48 hours after start of irradiation, all the proteins including BSA serving as a virus-stabilizer were found to be digested. This indicates that a tissue of virus proteins was destroyed by the action of the photocatalyst.

Example 13

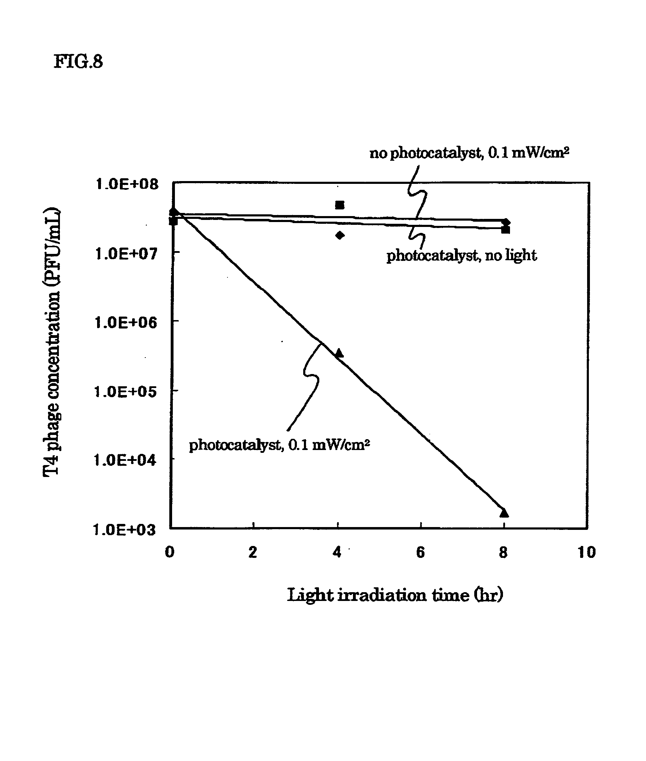

In Example 13, the "titanium oxide-coated glass plate" as employed in Example 12 was used as a sample. The procedure of Example 1 was repeated, except that a T4 phage solution was used instead of the Qβ phage solution, and that the UV light intensity of the light source was adjusted to 0.1 mW/cm$^2$, in "Anti-phage performance test" described in Example 1.
The results are shown in Fig. 8, indicated with "photocatalyst, 0.1 mW/cm$^2$."

Comparative Example 15

The procedure of Example 13 was repeated, except that the sample was not irradiated with light but was placed in the dark in "Anti-phage performance test." The results are shown in Fig. 8, indicated with "photocatalyst, no light."

Comparative Example 16

The procedure of Example 13 was repeated, except that the titanium oxide was not applied onto the glass plate serving as a sample. The results are shown in Fig. 8, indicated with "no photocatalyst, 0.1 mW/cm$^2$."
As is clear from Fig. 8, T4 phage was also inactivated only in the presence of the photocatalyst and under irradiation with light.

Industrial Applicability

According to the method of the present invention, viruses including influenza viruses and bacteriophages can be effectively inactivated through employment of a UV-beam-responsive or visible-light-responsive photocatalyst material, particularly a visible-light-responsive photocatalyst material under irradiation with light, particularly irradiation with light including visible light of a wavelength of 400 to 530 nm.

**Claims**

1. A method for inactivating a virus which is in contact with a photocatalyst material, the method comprising irradiating the photocatalyst material with light from a light source.

2. A virus inactivation method according to claim 1, wherein membrane protein in the virus which is in contact with the photocatalyst material is partially destroyed.

3. A virus inactivation method according to claim 1 or 2, wherein the photocatalyst material contains titanium oxide, and the light from the light source includes ultraviolet light having a wavelength of 350 to 400 nm.

4. A virus inactivation method according to claim 3, wherein the photocatalyst material is in the form of coating film, and the coating film contains titanium oxide in an amount of 10 mg/m$^2$ to 10 g/m$^2$.

5. A virus inactivation method according to claim 3 or 4, wherein the light source provides light having a wavelength of 350 to 400 nm and is any of sunlight, a black fluorescent lamp, an LED, and an organic EL.

6. A virus inactivation method according to claim 1 or 2, wherein the photocatalyst material contains a visible-light-responsive photocatalyst material, and the light from the light source includes visible light having a wavelength of 400 to 530 nm.

7. A virus inactivation method according to claim 6, wherein the visible-light-responsive photocatalyst material comprises, in combination, (A-1) a copper compound and/or an iron compound, and (B) at least one member selected from among tungsten oxide, titanium oxide, and titanium oxide having a conduction band controlled through doping.

8. A virus inactivation method according to claim 6, wherein the visible-light-responsive photocatalyst material comprises, in combination, (A-2) any of platinum, palladium, rhodium, and ruthenium, or a mixture of two or more species thereof, and (B) at least one member selected from among tungsten oxide, titanium oxide, and titanium oxide having a conduction band controlled through doping.

9. A virus inactivation method according to claim 6, wherein the photocatalyst material is in the form of coating film, and the coating film has a visible-light-responsive photocatalyst material content of 100 mg/m$^2$ to 20 g/m$^2$.

10. A virus inactivation method according to claim 5, wherein the light source provides light having a wavelength of 400 to 530 nm and is any of sunlight, a fluorescent lamp, an LED, and an organic EL.

11. A virus inactivation method according to claim 1 or 2, wherein the virus is an influenza virus.

12. A virus inactivation method according to claim 1 or 2, wherein the virus is a bacteriophage.

13. An article provided with an anti-viral property, comprising a visible-light-responsive photocatalyst material deposited on a surface thereof.

14. An article provided with an anti-viral property according to claim 13, wherein the visible-light-responsive photocatalyst material comprises, in combination, (A) a copper compound and/or an iron compound, and (B) at least one member selected from among tungsten oxide, titanium oxide, and titanium oxide having a conduction band controlled through doping.

FIG.1

FIG.2

FIG.3

FIG.4

FIG.5

FIG.6

FIG.7

FIG.8

**INTERNATIONAL SEARCH REPORT**

| International application No. |
| --- |
| PCT/JP2010/068333 |

A. CLASSIFICATION OF SUBJECT MATTER
*A61L2/16*(2006.01)i, *A61L9/00*(2006.01)i, *A61L9/01*(2006.01)i, *A61L9/18*
(2006.01)i, *A61L9/20*(2006.01)i, *B01J35/02*(2006.01)i

According to International Patent Classification (IPC) or to both national classification and IPC

B. FIELDS SEARCHED

Minimum documentation searched (classification system followed by classification symbols)
A61L2/00-2/28, A61L9/00-9/22, B01J21/00-38/74

Documentation searched other than minimum documentation to the extent that such documents are included in the fields searched
Jitsuyo Shinan Koho        1922-1996   Jitsuyo Shinan Toroku Koho    1996-2011
Kokai Jitsuyo Shinan Koho  1971-2011   Toroku Jitsuyo Shinan Koho    1994-2011

Electronic data base consulted during the international search (name of data base and, where practicable, search terms used)
WPI, JSTPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| X | Kazuhito HASHIMOTO et al., "Hikari Shokubai Gijutsu no Kanosei", Clean Technology, 10 June 2009 (10.06.2009), vol.19, no.6, pages 1 to 5 | 1-14 |
| X | Ryuichi NAKANO et al., "Sanka Titanium Hikari Shokubai ni yoru Influenza Virus no Fukatsuka Koka", The Society for Antibacterial and Antifungal Agents, Japan Nenji Taikai Yoshishu, 14 September 2009 (14.09.2009), vol.36th, page 190 | 1-14 |
| X | Yoshinobu KUBOTA, "Hikari Shokubai no Ko-virus Kassei ni Tsuite", CSJ: The Chemical Society of Japan Koen Yokoshu, 2009.03, vol.89th, no.1, page 318 | 1-14 |

[X] Further documents are listed in the continuation of Box C.  [ ] See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 14 January, 2011 (14.01.11) | 25 January, 2011 (25.01.11) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

| | International application No. |
|---|---|
| | PCT/JP2010/068333 |

C (Continuation). DOCUMENTS CONSIDERED TO BE RELEVANT

| Category* | Citation of document, with indication, where appropriate, of the relevant passages | Relevant to claim No. |
|---|---|---|
| X | JP 2008-119312 A (Taiyo Kagaku Co., Ltd.), 29 May 2008 (29.05.2008), claims; paragraphs [0011], [0012]; examples; drawings (Family: none) | 1-14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008149312 A
- JP 3649241 B
- JP 4240505 B
- JP 4240508 B
- JP 2008119312 A

**Non-patent literature cited in the description**

- **IRIE et al.** Bulletin Photocatalyst. April 2009, vol. 28, 4
- Bulletin Photocatalyst. July 2009, vol. 29, 38
- **K. OBATA et al.** *Chemical Physics,* 2007, vol. 339, 124-132